(19) Europäisches Patentamt

European Patent Office

Office européen des brevets

(11) **EP 1 069 098 B1**

(12) **EUROPÄISCHE PATENTSCHRIFT**

(45) Veröffentlichungstag und Bekanntmachung des Hinweises auf die Patenterteilung:
**04.06.2003   Patentblatt 2003/23**

(51) Int Cl.⁷: **C07C 2/46**

(21) Anmeldenummer: **00114749.5**

(22) Anmeldetag: **08.07.2000**

(54) **Verfahren zur Herstellung von Cyclododecatrienen mit Rückführung des Katalysators**

Process for the synthesis of cyclododecatrienes with recycling of the catalyst

Procédé pour la préparation de cyclododécatriènes avec recyclage du catalyseur

(84) Benannte Vertragsstaaten:
**AT BE CH CY DE DK ES FI FR GB GR IE IT LI LU MC NL PT SE**

(30) Priorität: **13.07.1999   DE 19932577**
             **21.01.2000   DE 10002460**

(43) Veröffentlichungstag der Anmeldung:
**17.01.2001   Patentblatt 2001/03**

(73) Patentinhaber: **Degussa AG**
**40474 Düsseldorf (DE)**

(72) Erfinder:
• **Wilczok, Norbert**
**45481 Mühlheim (DE)**
• **Wiedenbusch, Peter**
**45772 Marl (DE)**
• **Schiffer, Thomas, Dr.**
**45721 Haltern (DE)**

(56) Entgegenhaltungen:
**DE-A- 2 825 341          DE-B- 1 283 836
US-A- 3 723 553          US-A- 4 270 016**

## Beschreibung

Die Erfindung betrifft ein vorzugsweise kontinuierliches Verfahren zur Herstellung von Cyclododecatrienen (CDT) an einem insbesondere Nickel oder Titan enthaltenden Katalysatorsystem mit destillativer Abtrennung der Roh-Cyclododecatriene und Rückführung des Katalysators.

Die Synthese von CDT ausgehend von 1,3-Butadien ist sowohl mit homogenen als auch mit heterogenen Übergangsmetallkatalysatoren untersucht worden. Bei heterogenen Katalysatorsystemen wird ein Übergangsmetallkomplex typischerweise über einen Brückenliganden an einen polymeren Träger gebunden (U. Schuchardt, J. Mol. Catal. 29 (1985) 145). Solche Festbettsysteme haben den gravierenden Nachteil, daß der Brückenligand im Wettbewerb zu Butadien, CDT und olefinischen Zwischenstufen um eine freie Koordinationsstelle am Katalysator tritt. Dadurch sinkt die Umsatzrate des Festbettkatalysators erheblich und der Anteil dimerer und/oder oligomerer Nebenprodukte nimmt in der Regel zu. Zudem kann CDT den Brückenliganden vom Übergangsmetall verdrängen. Dadurch wird das Metallatom aus dem Festbett herausgelöst. Der Festbettkatalysator verliert seine aktiven Zentren, seine katalytische Aktivität nimmt ab.

Bei der großtechnischen Umsetzung der Synthese haben sich daher homogene Katalysatoren gegenüber Festbettkatalysatoren allgemein durchgesetzt. Die Vorteile homogener Katalysatoren liegen vor allem in sehr guten Raum-Zeit-Ausbeuten und in hohen Selektivitäten zugunsten von CDT. Von den in der Literatur (G. Wilke, Angew. Chemie 69 (1957) 397; H. Breil, P. Heimbach, M. Kröner, H. Müller, G. Wilke, Makromolekulare Chemie 69 (1963) 18; G. Wilke, M. Kröner, Angew. Chemie 71 (1959) 574) bekannten Übergangsmetallen werden insbesondere Titan-, Chrom- und Nickelverbindungen verwendet. Katalytisch aktiv sind diese Übergangsmetalle in Form von metallorganischen Komplexen, in denen das Zentralatom die Oxidationsstufe 0 besitzt. Typischerweise werden diese metallorganischen Komplexe aus einem Übergangsmetallsalz und einem Reduktionsmittel hergestellt. Zur Reduktion wird in der Regel eine metallorganische Verbindung aus der 1. - 3. Gruppe des Periodensystems verwendet. Für die in der industriellen Anwendung weit verbreiteten Titankatalysatoren hat sich insbesondere die Umsetzung von Titantetrachlorid oder Titanacetylacetonat mit einem Aluminiumorganyl als günstig erwiesen (US 3 878 258, US 3 655 795, beide E. I. du Pont de Nemours; DE 30 21 840, DE 30 21 791, beide Chemische Werke Hüls), doch wurden auch zahlreiche weitere Titansalze und Reduktionsmittel als Ausgangsverbindungen beschrieben (zum Beispiel DE 19 46 062, Mitsubishi Petrochemical Co.; US 3 644 548, Asahi Chemical Industry).

Bei der großtechnischen Synthese von CDT mit einem homogenen Katalysator erfolgt die Reaktion üblicherweise in einem kontinuierlichen Prozeß mit einem oder mehreren Rührkesseln. Ein Teil der Reaktionsmischung wird dabei kontinuierlich aus den Reaktoren ausgeschleust. Bei der Aufarbeitung wird nicht umgesetztes Edukt zurückgewonnen und zusammen mit frischem Butadien in den Reaktionsprozeß zurückgeführt. Mit dem Reaktoraustrag wird auch ein Teil des Katalysators ausgeschleust. Die Konzentration an Katalysator im Reaktor wird deshalb üblicherweise durch eine kontinuierliche Zugabe frischer Katalysatorbestandteile konstant gehalten.

Vor der Aufarbeitung des Reaktoraustrags muß der ausgeschleuste Katalysator zersetzt werden. Hierfür eignen sich diverse polare Lösemittel sehr gut. Neben Wasser verwenden Ube Industries beispielsweise eine Ammoniumhydroxidlösung (JP 05 070 377, JP 06 254 398, beide Ube Industries, zitiert nach CA 119:72275 und CA 121: 303571). Auch diverse Alkohole (JP 7 625 439, JP 7 625 396, beide Agency of Industrial Sciences and Technology, Japan, zitiert nach CA 86:17321 und CA 86:17322), eignen sich dafür, insbesondere wird Methanol (JP 7 442 496, Toyo Soda Co., zitiert nach CA 82:139521) und methanolische Salzsäure (DE 19 42 729, Mitsubishi Petrochemical Co.) verwendet.

Die Zersetzung des Katalysators gelingt auch mit Hilfe von Aceton (JP 43 013 451, Toyo Rayon, zitiert nach CA 70:77450) oder mit einer Suspension von Calciumoxid in Wasser (NL 6 603 264, Shell Int. Research Maatschappij N. V.). Ube Industries merken an, daß sich das gebildete CDT nur unvollständig gewinnen läßt, wenn Wasser zur Zersetzung des Katalysators verwendet wird. Die CDT-Ausbeute läßt sich aber verbessern, wenn wäßriges Tetrahydrofuran eingesetzt wird (JP 05 070 377, zitiert nach CA 119:72275).

Alle erwähnten Beispiele zur homogen katalysierten CDT-Synthese nehmen bei der Aufarbeitung eine Zersetzung des Katalysatorsystems in Kauf. Aufgrund der hohen Umsatzrate und Selektivität des Katalysators sind die benötigten Katalysatormengen im Vergleich zur gebildeten Menge CDT zwar gering, dennoch ist eine Alternative in der Aufarbeitung verbunden mit einer Rückführung des aktiven Katalysators wünschenswert. Dies gilt wegen der bei der Aufarbeitung anfallenden Schwermetallfrachten im Abwasser insbesondere für die beiden Übergangsmetalle Chrom und Nickel.

Die großtechnische Cyclodimerisierung von 1,3-Butadien zu Cyclooctadien (COD) erfolgt in flüssiger Phase an einem Nickel(0)komplex. Der homogene Katalysator besteht dabei aus dem Übergangsmetall und einem sperrigen Donorliganden, typischerweise ein Phosphin oder ein Phosphit. Von diesem Katalysatorsystem ist bekannt, daß es sich partiell zurückgewinnen und dadurch mehrfach einsetzen läßt.

Dazu wird der Reaktoraustrag üblicherweise in einer fraktionierten Destillation aufgearbeitet. Dabei werden nicht umgesetztes Edukt, COD und leicht siedende Nebenprodukte abgetrennt. Es bleibt ein höher siedender Rückstand übrig, in dem der Katalysator gelöst vorliegt. Dieser Rückstand wird in den Reaktionsprozeß zurückgefahren und der Katalysator zur erneuten Butadiendimerisierung eingesetzt. Erst nach mehreren Zyklen hat sich der Anteil an Hochsiedern so stark in der Reaktionsmischung angereichert, daß der Katalysator mit der Hochsiederfraktion ausgeschleust und verworfen werden muß.

Bei CDT (1,5,9-Cyclododecatrien), das in trans-trans-trans-, cis-trans-trans- und ciscis-trans-Isomerenform entsteht, ist eine Rückführung des Katalysators im großtechnischen Maßstab bisher noch nicht beschrieben worden. Bekannt sind lediglich einige Beispiele mit absatzweise durchgeführter Fahrweise, in denen eine mehrfache Verwendung des Katalysators im Labormaßstab versucht wurde. So wird in DE 30 21 791 A1 beschrieben, daß der Katalysator zunächst an Aktivkohle adsorbiert und später zusammen mit der Aktivkohle abfiltriert wird. Dieses Verfahren hat sich jedoch in der Praxis nicht bewährt, da sich der Katalysator auf diese Weise nur teilweise zurückgewinnen läßt.

In DE 12 83 836, Beispiel 3, wird beschrieben, daß sich der Ni(0)-COD-Komplex nach der Reaktion in Gegenwart des Lösemittels Benzol im Sumpf der schwersiedenden Destillationsprodukte sammelt und noch eine Restaktivität gegenüber Butadien besitzt, so daß er noch einmal zur Cyclisierung eingesetzt werden kann. Es werden jedoch keinerlei weitere Angaben über Katalysatorrückführung gemacht.

Auch in Beispiel 7, DE-OS 28 25 341 wird erwähnt, daß sich der Katalysator nach der Reaktion in Gegenwart des Lösemittels und Moderators Dibenzylbenzol nach einer Destillation bei 80 °C und 0,5 Torr im Rückstand sammelte und dieser noch in zwei weiteren Ansätzen eingesetzt wurde. Nach dem dritten Ansatz verlor der Katalysator allerdings deutlich an Aktivität.

Aufgabe war es daher, für die CDT-Synthese ein Verfahren zu entwickeln, bei dem der Katalysator zurückgeführt und mehrmals verwendet werden kann. Überraschend wurde nun gefunden, daß Übergangsmetallkomplexe des CDT und des COD, insbesondere Ni(0)-Komplexe, sehr stabil sind und nach destillativem Abtrennen des Roh-CDT's und des größten Teils der Nebenprodukte (COD, Vinylcyclohexen VCH)) und nach Teilausschleusen der Hochsieder als aufkonzentrierte Katalysatorlösungen in den Prozeß zurückgeführt und erneut eingesetzt werden können.

Gegenstand der Erfindung ist daher ein Verfahren zur Herstellung von Cyclododecatrienen aus 1,3-Butadien in Gegenwart eines insbesondere Nickel oder Titan enthaltenden Katalysatorsystems, bei dem das Katalysatorsystem nach destillativer Abtrennung der Roh-Cyclododecatriene zu 50 bis 100 % zurückgeführt wird und nur der mit den Hochsiedern ausgeschleuste Katalysator durch frischen Katalysator ersetzt wird, wobei die Umsetzung von 1,3-Butadien in Gegenwart von Cyclooctadien und/oder Cyclododecatrien frei von systemfremden Lösemitteln durchgeführt wird.

Als Katalysatorausgangsmaterialien werden vorzugsweise handelsübliche Nickel(II)- bzw. Titan(IV)-haltige Verbindungen eingesetzt. Als Beispiel für eine Nickelverbindung sei das Nickelacetylacetonat und als Beispiel für eine Titanverbindung das Titantetrachlorid genannt.

Die Reaktion wird mit Katalysatorkonzentrationen zwischen 0,01 mmol/l und 40 mmol/l Katalysator, vorzugsweise zwischen 0,05 mmol/l und 10 mmol/l Katalysator (bezogen auf Nickel oder Titan) durchgeführt.

Als Verbindungen für die Katalysatoraktivierung werden metallorganische Verbindungen des Aluminiums verwendet. Als bevorzugte Verbindungen seien Ethoxydiethylaluminium und Ethylaluminiumsesquichlorid genannt.

Das Verhältnis von metallorganischer Aluminiumverbindung zu der Nickelverbindung wird so gewählt, daß das molare Verhältnis von Nickel zu Aluminium 1: 3 bis 1: 6 beträgt.

Im Falle des Titans beträgt das molare Verhältnis von Titan zu Aluminium 1 : 10 bis 1 : 40.

Die Reaktionstemperatur beträgt 60 °C bis 120 °C, vorzugsweise 70 °C bis 115 °C. Eine höhere Temperatur als 120 °C ist zu vermeiden, weil ein höherer Anteil von Nebenprodukten gebildet wird und es bei Temperaturen > 120 °C zur irreversiblen Schädigung des Katalysatorsystems kommen kann.

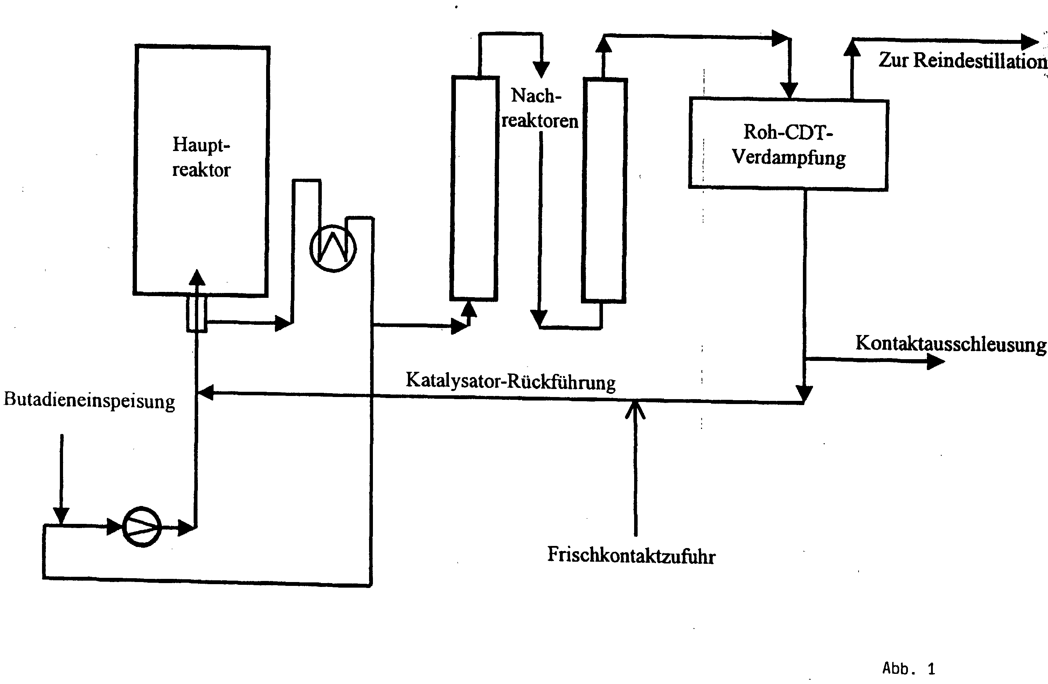

Die Rückführung des Katalysators (Kreisfahrweise) ist in der Abbildung 1 schematisch dargestellt. Das Reaktionsgemisch wird aus den Nachreaktoren kommend in den Vakuumbehälter (Roh-CDT-Verdampfung) entspannt und bei einer Temperatur von 90 °C bis 120 °C und einem Druck von 2 mbar bis 40 mbar in Reste unumgesetzten Butadiens, Roh-Cyclododecatriene (mit COD und VCH) und Hochsieder mit Katalysator destillativ getrennt. Das aus der Roh-CDT-Verdampfung abgeführte Destillat wird der Reindestillation zugeführt.

Nach eventueller Ausschleusung eines kleinen Teils des Rückstandes (Kontaktausschleusung), wird der Katalysator unter eventueller Zufuhr frischen Katalysators (Frischkontaktzufuhr) vor den Hauptreaktor zurückgeführt. Die Rückführung erfolgt zu 50 % bis 100 %, vorzugsweise zu 80 % bis 98 %, besonders bevorzugt zu 90 % bis 95 %.

Die erstmalige Füllung des Reaktorsystems kann zum Beispiel über die Leitung Butadieneinspeisung erfolgen.

Die Reaktion kann diskontinuierlich oder bevorzugt kontinuierlich durchgeführt werden.

Als Lösemittel für das Katalysatorsystem dienen die ohnehin im System vorhandenen Stoffe, also überwiegend Cyclooctadien und/oder Cyclododecatrien. Die Reaktion wird also frei von systemfremden Lösemitteln durchgeführt.

Beispiele

Die im folgenden beschriebenen Versuche wurden in einer Anlage mit einem Hauptreaktor von 20 m³ Inhalt und zwei in Reihe geschalteten Nachreaktoren von je 1,5 m³ Inhalt kontinuierlich durchgeführt.

Die Rückführung des Katalysators betrug ca. 95 %.

Es wurden gaschromatographische Analysen des Produktes nach Entspannungsverdampfung zur Reindestillation angefertigt. Zur Trennung wurde eine Kapillarsäule HP-20M (Carbowax 20M), Länge 50 m, Durchmesser 0,32 mm, Filmdicke 30 µm verwendet.

Aus den Analysen wurde die Selektivität berechnet.

Definition der Selektivität S am Beispiel des CDT:

$$S_{CDT} = \frac{\text{Summe CDT-Isomere}}{\Sigma \text{ COD; VCH; CDT-Isomere; Hochsieder}} \times 100 \text{ \%}$$

Die Selektivitäten der anderen Komponenten werden analog berechnet.

Beispiel 1:

Im Hauptreaktor wurden ca. 15 m³ Cyclooctadien-1,5 vorgelegt. Als Katalysator wurde Nickelacetylacetonat/Ethoxydiethylaluminium verwendet. Nach Einfahren der berechneten Katalysatormengen ($c_{Ni}$ = 6,7 mmol/l, $c_{Al}$ = 20 mmol/l) wurde der Reaktor auf 85 °C aufgeheizt und Butadien zunächst mit einer Last von 1 200 kg/h für 3,5 Stunden zur Auffüllung des Reaktors eingefahren. Danach wurde die Last auf 650 kg Butadien pro Stunde reduziert.

Der Gesamtumsatz an Butadien im Hauptreaktor betrug ca. 94 %. In den Nachreaktoren veränderte er sich kaum noch.

Die Gesamtselektivität für Cyclododecatrien lag bei 88,5 %.

Die Selektivitäten sind in der Tabelle I angegeben.

Tabelle I

| Selektivitäten | |
|---|---|
| | % |
| Cyclooctadien-1,5 | 4,0 |
| Vinylcyclohexen | 4,0 |
| Trans-trans-trans-Cyclododecatrien | 69,5 |
| Cis-cis-trans-Cyclododecatrien | 11,5 |
| Cis-trans-trans-Cyclododecatrien | 7,5 |
| Hochsieder | 3,5 |

Die Raum-Zeit-Ausbeute lag bei 5 - 9 kg CDT/h·m³.

Beispiel 2:

Alle 3 Reaktoren wurden mit Cyclooctadien und der berechneten Katalysatormenge ($c_{Ni}$ = 20 mmol/l, $c_{Al}$ = 60 mmol/l) gefüllt und auf 88 °C aufgeheizt. Nun wurde Butadien mit 1 200 kg/h für 30 Minuten eingefahren. Die Last wurde danach auf 500 kg Butadien pro Stunde reduziert.

Der Gesamtumsatz an Butadien im Hauptreaktor betrug 94,3 %. In den Nachreaktoren stieg er bis auf 96,3 % an.

Die Gesamtselektivität für Cyclododecatrien (trans-trans-trans, cis-cis-trans und cistrans-trans) lag bei 84 %.

Die Selektivitäten sind in der Tabelle II angegeben.

Tabelle II

| Selektivitäten | |
|---|---|
| | % |
| Cyclooctadien-1,5 | 4,0 |
| Vinylcyclohexen | 4,0 |
| Trans-trans-trans-Cyclododecatrien | 64,5 |
| Cis-cis-trans-Cyclododecatrien | 11,5 |
| Cis-trans-trans-Cyclododecatrien | 8,0 |
| Hochsieder | 8,0 |

Die Raum-Zeit-Ausbeute lag bei 14 - 18 kg CDT/h·m$^3$.

Beispiel 3:

Der Versuch wurde wie Versuch 2 durchgeführt Die Temperatur betrug jedoch 93 °C.

Der Gesamtumsatz an Butadien im Hauptreaktor betrug 96,2 %. In den Nachreaktoren stieg er bis auf 98 % an.

Die Gesamtselektivität für Cyclododecatrien (trans-trans-trans, cis-cis-trans und cistrans-trans) lag bei 90 %.

Die Selektitiväten sind in der Tabelle III angegeben.

Tabelle III

| Selektivitäten | |
|---|---|
| | % |
| Cyclooctadien-1,5 | 4,5 |
| Vinylcyclohexen | 1,5 |
| Trans-trans-trans-Cyclododecatrien | 59,5 |
| Cis-cis-trans-Cyclododecatrien | 16,5 |
| Cis-trans-trans-Cyclododecatrien | 14,0 |
| Hochsieder | 4,0 |

Die Raum-Zeit-Ausbeute lag bei 14 - 18 kg CDT/h·m$^3$.

Beispiel 4:

Der Versuch wurde wie Versuch 2 durchgeführt. Die Temperatur betrug jedoch 98 °C.

Der Gesamtumsatz an Butadien im Hauptreaktor betrug 98,3 %. In den Nachreaktoren stieg er bis auf 99,1 % an.

Die Gesamtselektivität für Cyclododecatrien (trans-trans-trans, cis-cis-trans und cistrans-trans) lag bei 90,5 %.

Die Selektivitäten sind in der Tabelle IV angegeben.

Tabelle IV

| Selektivitäten | |
|---|---|
| | % |
| Cyclooctadien-1,5 | 4,0 |
| Vinylcyclohexen | 1,5 |
| Trans-trans-trans-Cyclododecatrien | 60,0 |
| Cis-cis-trans-Cyclododecatrien | 16,5 |

EP 1 069 098 B1

Tabelle IV   (fortgesetzt)

| Selektivitäten | |
| --- | --- |
| | % |
| Cis-trans-trans-Cyclododecatrien | 14,0 |
| Hochsieder | 4,0 |

Die Raum-Zeit-Ausbeute lag bei 14-18 kg CDT/h·m$^3$.

Beispiel 5:

Im Hauptreaktor wurden 15 m$^3$ 99,8 %iges Cyclododecatrien vorgelegt. Als Katalysatorsystem wurde Titantetrachlorid und Ethylaluminiumsesquichlorid verwendet. Nach Einfahren der Katalysatormengen ($C_{Ti}$ = 0,05 mmol/l, $C_{Al}$ = 0,2 mmol/l, wurde der Reaktor auf 70 °C aufgeheizt und 1 200 kg/h Butadien eingeleitet. Der Gesamtumsatz an Butadien betrug 99 %.
Die Selektivitäten sind in Tabelle V angegeben.

Tabelle V

| Selektivitäten | |
| --- | --- |
| | % |
| Cyclooctadien-1,5 | 1,6 |
| Vinylcyclohexen | 1,2 |
| Trans-trans-trans-Cyciododecatrien | 0,7 |
| Cis-cis-trans-Cyclododecatrien | 0,1 |
| Cis-trans-trans-Cyclododecatrien | 93,4 |
| Hochsieder | 3,0 |

## Patentansprüche

1. Verfahren zur Herstellung von Cyclododecatrienen aus 1,3-Butadien in Gegenwart eines Katalysatorsystems, welches nach der destillativen Abtrennung der Rohcyclododecatriene zu 50 bis 100 % zurückgeführt wird,
**dadurch gekennzeichnet,**
**dass** die Umsetzung von 1,3-Butadien in Gegenwart von Cyclooctadien und/oder Cyclododecatrien frei von systemfremden Lösemitteln durchgeführt wird.

2. Verfahren nach Anspruch 1,
**dadurch gekennzeichnet, daß** das Katalysatorsystem zu 80 % bis 98 % zurückgeführt wird.

3. Verfahren nach Anspruch 1,
**dadurch gekennzeichnet, daß** das Katalysatorsystem zu 90 % bis 95 % zurückgeführt wird.

4. Verfahren nach einem der Ansprüche 1 bis 3,
**dadurch gekennzeichnet, daß** das Katalysatorsystem Nickelverbindungen enthält.

5. Verfahren nach Anspruch 4,
**dadurch gekennzeichnet, daß** die Ausgangs-Nickelverbindung für das Katalysatorsystem Nickelacetylacetonat ist.

6. Verfahren nach einem der Ansprüche 1 bis 3,
**dadurch gekennzeichnet, daß** das Katalysatorsystem Titanverbindungen enthält.

7. Verfahren nach Anspruch 6,

**dadurch gekennzeichnet, daß** die Ausgangs-Titanverbindung für das Katalysatorsystem Titantetrachlorid ist.

8. Verfahren nach einem der Ansprüch 1 bis 7,
**dadurch gekennzeichnet, daß** das Verfahren kontinuierlich durchgeführt wird.

9. Verfahren nach einem der Ansprüche 1 bis 8,
**dadurch gekennzeichnet, daß** die Reaktion bei Temperaturen zwischen 60 °C und 120 °C durchgeführt wird.

10. Verfahren nach einem der Ansprüche 1 bis 9,
**dadurch gekennzeichnet, daß** das Gemisch aus Roh-Cyclododecatrien und Katalysatorsystem sowie Nebenprodukten in den Vakuumbehälter entspannt und bei einer Temperatur von 90 °C bis 120 °C und einem Druck von 2 bis 40 mbar destillativ getrennt wird.

11. Verfahren nach einem der Ansprüche 1 bis 10,
**dadurch gekennzeichnet, daß** im Prozeß entstehende Hochsieder aus dem laufenden Prozeß ausgeschleust werden und der mit den Hochsiedern ausgeschleuste Katalysator durch die entsprechende Menge an frischem Katalysator ersetzt wird.

12. Verfahren nach einem der Ansprüche 1 bis 11,
**dadurch gekennzeichnet, daß** die Katalysatorkonzentration so gewählt wird, daß 0,01 mmol/l bis 40 mmol/l Nickel oder Titan im Reaktionssystem enthalten sind.

**Claims**

1. A process for preparing cyclododecatrienes from 1,3-butadiene in the presence of a catalyst system of which from 50 to 100% is recycled after the crude cyclododecatrienes have been separated off by distillation, **characterized in that** the reaction of 1,3-butadiene is carried out in the presence of cyclooctadiene and/or cyclododecatriene in the absence of solvents extraneous to the system.

2. A process according to claim 1, **characterized in that** from 80% to 98% of the catalyst system is recycled.

3. A process according to claim 1, **characterized in that** from 90% to 95% of the catalyst system is recycled.

4. A process according to any one of claims 1 to 3, **characterized in that** the catalyst system comprises nickel compounds.

5. A process according to claim 4, **characterized in that** the starting nickel compound for the catalyst system is nickel acetylacetonate.

6. A process according to any one of claims 1 to 3, **characterized in that** the catalyst system comprises titanium compounds.

7. A process according to claim 6, **characterized in that** the starting titanium compound for the catalyst system is titanium tetrachloride.

8. A process according to any one of claims 1 to 7, **characterized in that** the process is carried out continuously.

9. A process according to any one of claims 1 to 8, **characterized in that** the reaction is carried out at temperatures of from 60°C to 120°C.

10. A process according to any one of claims 1 to 9, **characterized in that** the mixture of crude cyclododecatriene and catalyst system together with by-products is depressurized in a vacuum vessel and is fractionated by distillation at a temperature of from 90°C to 120°C and a pressure of from 2 to 40 mbar.

11. A process according to any one of claims 1 to 10, **characterized in that** high boilers formed in the process are discharged from the process during operation and the catalyst discharged together with the high boilers is replaced by the corresponding amount of fresh catalyst.

**12.** A process according to any one of claims 1 to 11, **characterized in that** the catalyst concentration is chosen so that from 0.01 mmol/l to 40 mmol/l of nickel or titanium is present in the reaction system.

**Revendications**

**1.** Procédé de préparation de cyclododécatriène à partir de 1,3-butadiène en présence d'un système de catalyseurs qui est recyclé pour 50 à 100 % après la séparation par distillation du cyclododécatriène brut,
**caractérisé en ce que**
la réaction du 1,3-butadiène en présence de cyclooctadiène et/ou de cyclododécatriène est effectuée sans solvant extérieur au système.

**2.** Procédé selon la revendication 1,
**caractérisé en ce que**
le système de catalyseurs est recyclé pour 80 à 98 %.

**3.** Procédé selon la revendication 1,
**caractérisé en ce que**
le système de catalyseurs est recyclé pour 90 à 95 %.

**4.** Procédé selon l'une quelconque des revendications 1 à 3,
**caractérisé en ce que**
le système de catalyseurs contient des composés du nickel.

**5.** Procédé selon la revendication 4,
**caractérisé en ce que**
le composé du nickel de départ pour le système de catalyseurs est l'acétylacétonate de nickel.

**6.** Procédé selon l'une quelconque des revendications 1 à 3,
**caractérisé en ce que**
le système de catalyseurs contient des composés du titane.

**7.** Procédé selon la revendication 6,
**caractérisé en ce que**
le composé du titane de départ pour le système de catalyseurs est le tétrachlorure de titane.

**8.** Procédé selon l'une quelconque des revendications 1 à 7,
**caractérisé en ce que**
le procédé est exécuté en continu.

**9.** Procédé selon l'une quelconque des revendications 1 à 8,
**caractérisé en ce que**
la réaction est effectuée à des températures comprises entre 60°C et 120°C.

**10.** Procédé selon l'une quelconque des revendications 1 à 9,
**caractérisé en ce que**
le mélange à base de cyclododécatriène brut et le système de catalyseurs ainsi que de sous-produits, est détendu dans un récipient de vide et est séparé par distillation à une température de 90°C à 120°C et sous une pression de 2 à 40 mbars.

**11.** Procédé selon l'une quelconque des revendications 1 à 10,
**caractérisé en ce que**
les fractions à haut point d'ébullition formées dans le processus, sont évacuées du processus en cours, et le catalyseur évacué avec les fractions à haut point d'ébullition est remplacé par la quantité correspondante de catalyseur frais.

**12.** Procédé selon l'une quelconque des revendications 1 à 11,
**caractérisé en ce que**

la concentration en catalyseur est choisie de sorte que de 0,01 mmol/l à 40 mmol/l de nickel ou de titane soient présents dans le système de réaction.

Abb. 1

EP 1 069 098 B1